# EUROPEAN PATENT SPECIFICATION

(11) **EP 1 948 063 B1**
(45) Date of publication and mention of the grant of the patent: **18.08.2010**
(21) Application number: 06844384.5
(22) Date of filing: 16.11.2006
(51) Int. Cl.: A61B 19/00

(54) **MARKER FOR MARKING AN AREA IN BODY TISSUE**
MARKER ZUR MARKIERUNG EINES BEREICHS IN EINEM KÖRPERGEWEBE
MARQUEUR PERMETTANT DE MARQUER UNE ZONE SUR UN TISSU CORPOREL

(30) Priority: 16.11.2005 US 737178 P
(43) Date of publication of application: 30.07.2008
(73) Proprietor: William Cook Europe ApS, 4632 Bjaeverskov (DK); Cook Incorporated, Bloomington, Indiana 47402-0489 (US)
(72) Inventor: HANSEN, Palle, M., DK-4632 Bjaeverskov (DK)
(74) Representative: Garratt, Peter Douglas
(86) International application number: PCT/US2006/044485
(87) International publication number: WO 2007/059276

(56) References cited:
- WO-A-92/12678
- US-A1- 2004 077 971
- US-A1- 2004 168 692

## Description

### Technical Field

The present invention relates to a marker for marking an area in body tissue.

### Background of the Invention

It is often desirable and frequently necessary to sample or remove a portion of tissue from humans and other animals, particularly when diagnosing and treating patients with cancerous tumours, pre-malignant conditions, and other diseases or disorders.

Typically, in the case of cancer, particularly cancer of the breast, there is great emphasis on early detection, diagnosis and treatment through the use of screening means, such as physical examination, and particularly mammography, which is capable of detecting very small abnormalities, often non-palpable. When the physician by means of a mammogram or other screening modality such as ultrasound establishes that suspicious circumstances exist, a biopsy must be performed to capture tissue for a definitive diagnosis as to whether the suspicious lesion is cancerous. Biopsy may be performed by an open or percutaneous technique. Open biopsy is a surgical procedure using a scalpel and involving direct vision of the target area, for removing the entire mass (excisional biopsy) or part of the mass (incisional biopsy). In the treatment of breast cancer, a surgical procedure that uses a scalpel and removes no less than the entire mass is usually applied.

When an open surgical biopsy or treatment procedure is indicated, current practice dictates the use of lesion localization needles and markers, commonly referred to as "localization wires", for use in localizing or marking non-palpable lesions and tumours within the body. These devices generally comprise a hypodermic needle or cannula, which is inserted into the body under local anaesthesia into the lesion or tissue of interest. The wire marker, or localization wire, is then passed through the cannula and extends through the lesion of interest so that the distal end thereof is anchored beyond the lesion. Thus, the lesion is marked for subsequent surgical procedures such as excision or biopsy. The anchoring procedure is typically accomplished by means of a mechanical structure disposed at the distal end of the wire marker, such as a barb, hook, or the like, which is attached to surrounding tissue. After marking the lesion with the wire marker, the cannula is usually removed from the body, leaving the marker in place and extending from the body of the patient for subsequent use by the surgeon during procedure for identifying the lesion location.

However, the part of the marker extending from the body may unintentionally be touched, causing discomfort and even pain to the patient. It would, therefore, be desirable to develop a marker, where the risk of touching the part of the marker extending from the body is substantially eliminated in order to minimize discomfort and pain.

### Summary of the Invention

An object of the present invention is to provide a marker for marking an area in body tissues by means of which the risk of unintentionally touching causing discomfort and pain is minimized.

This object is met by the invention as defined in the claims.

The present invention provides a marker for use in body tissue, which highly reduces the risk of unintentionally touching the part of the marker extending from the body. It thereby also reduces the risk of causing unnecessary pain and discomfort to the patient wearing the marker according to the invention. Moreover, as a further advantage, the possibility that a push or blow on the part of the marker extending from the body may dislocate the marker in the body tissue is substantially eliminated.

Consequently, a highly body-friendly marker is obtained which also has excellent properties in respect of guiding the surgeon to the lesion location. Due to its body-friendly properties the marker is particularly suitable for marking lesions in the breast region.

In a first aspect, the invention relates to a marker for marking an area in body tissue. The marker is substantially rod-shaped with a first part having anchoring means intended to anchor the first part in body tissue in a body and a second part intended to substantially extend from the body while said first part is anchored in body tissue. The marker is preferably made from a super-elastic NiTi alloy, and the bending stiffness of the first part is at least twice as high as the bending stiffness of the second part when the first part is anchored in body tissue.

As it will be understood, the bending stiffness expresses the tendency of the rod or tube to bend when exposed to forces perpendicular to the longitudinal direction of the rod or tube. The less the tendency to bend the more stiff or rigid the rod or tube will be.

The first part of the marker should have a bending stiffness, which is sufficient to allow for a secure anchoring in body tissue. The bending stiffness of the second part should not be more than half of the bending stiffness of the first part. In this way, the second part, which is intended to substantially extend from the body, will be experienced as softer, and due to this "softness", it is substantially unable to transfer the effect of pushes and blows to the first part of the marker. Thereby, the marker will be more comfortable for the patient.

Moreover, for improving the production of the marker, it is an advantage that the marker can be made in one single material. For this purpose the super elastic NiTi alloys are highly suitable as they can have different material properties in different parts of the material depending on the treatment of the different parts. Thus, it is preferred that the marker is manufactured from a nitinol tube. Nitinol tubes made from suitable NiTi alloys (which may be subjected to further treatment) can be obtained as standard product from various suppliers. The treatment may e.g. be heat treatment or mechanical treatment.

For the purpose of making the marker more comfortable, it is preferred that the bending stiffness of the first part is at least four times, preferably at least eight times as high as the bending stiffness of the second part when the first part is anchored in body tissue.

For an even more comfortable marker, it is preferred that the bending stiffness of the first part is at least twenty times as high, preferably forty times as high as the bending stiffness of the second part when the first part is anchored in body tissue.

If the bending stiffness of the first part is at least fifty times as high, preferably eighty times as high, more preferably one hundred times as high as the bending stiffness of the second part, extremely comfortable and body-friendly markers can be obtained.

In a preferred embodiment of a marker according to the invention, the super elastic material is selected from the group consisting of super elastic alloys based on nickel and titanium. The super elastic alloy may be a pure NiTi alloy, or it may comprise minor amounts or traces of Cu, Al, Ag, Au, Zn, O, C, and N. The amount of the latter constituents does preferably not exceed 2 weight-%, more preferably the constituents do not exceed 1 weight-% of the alloy.

In a particularly preferred embodiment of the marker, the super elastic material is the Ni-Ti alloy, nitinol, which has excellent properties in respect of forming a marker according to the invention. Moreover, nitinol is highly acceptable for use in surgical devices. For most purposes it is preferred that the super elastic material has a transition temperature below 35°C. The transition temperature is the temperature where super elastic material undergoes a phase transformation, from one phase to another, which phases have different properties in relation to hardness, stiffness, elasticity etc. In case of nitinol, the phase transformation will be a change in phase from a martensitic phase at low temperatures to an austenitic phase at high temperatures. Nitinol in martensitic phase is less rigid or softer than nitinol in the austenitic phase. Thus, when applying nitinol with a transition temperature below 35°C, the first part of the marker will be in a austenitic phase when anchored in body tissue having a temperature of approximately 37°C, while the second part extending from the body will be at room-temperature, e.g. 23°C, and in the martensitic phase in which the alloy is softer.

In an alternatively preferred embodiment of the marker according to the invention the super elastic material of the first part has a transition temperature below 35°C, and the super elastic material of the second part has a transition temperature below 50°C. Thus, in case of nitinol, the second part will not transform from the martensitic phase to the austenitic phase at temperatures below 50°C, but will remain in the softer martensitic phase. Therefore, the marker is useable at temperatures exceeding normal room temperatures, e.g. 25-35°C (in operating rooms the temperature may be quite high due to heating caused by lamps and other equipment), while still maintaining the second part in a softer phase. Hereby, another advantage of the NiTi alloy can be utilized as the same piece of the alloy material can be designed with different properties in different parts of the alloy material.

Although the first part of the marker may be equipped with a large number of different anchoring means, e.g. crossing bars or expanding members, at least part of the first part of the marker is, however, barbed in a preferred embodiment. One or more barbs in the first part of the marker provide for a stable anchoring of the marker. The barbs may be provided by laser-cutting the rod or by twisting one or more threads around the first part of the rod. The threads may be made from nitinol or stainless steel or similar tissue-friendly material.

Preferably, the marker has a length in the range of 2 to 30 cm. Moreover, it is preferred that the first part of the marker has a length in the range of 1 to 14 cm, and that the marker has a circular cross-section with a diameter in the range of 0.25 to 1.5 mm. In this way, the marker is suitable to provide satisfactory guidance for surgeons in the surgical applications as mentioned above.

In a preferred embodiment the marker according to the invention comprise a hollow rod. The use of a hollow rod can save material and, furthermore, be advantageous in respect of providing desired stiffness or softness to the rod as compared to a solid rod.

Furthermore, the invention relates to a method for producing a marker for marking an area in body tissue, which method comprises the steps of:
providing a rod-shaped member comprising a super elastic material,
dividing said rod-shaped member into a first part and a second part, optionally providing said first part with anchoring means,
and subjecting the first part and/or the second part to a treatment to provide a bending stiffness of said first part which is at least twice as high as the bending stiffness of said second part, when said first part is held at a temperature in the range of 35-40°C.

According to the method, preferably the first part and/or the second part is subjected to a treatment so that the bending stiffness of the first part is at least ten times as high, preferably twenty times as high as the bending stiffness of the second part, when said first part is held at a temperature in the range of 35-40°C.

In this manner, it is possible to obtain a marker, in which the part intended to be anchored in body tissue has sufficient bending stiffness to secure a satisfactory anchoring, whereas the part intended to extend from the body has sufficient low bending stiffness to provide a soft appearance.

Suitable treatments to obtain the differences in the bending stiffness are described later.

Preferably, the super elastic material is selected from the group consisting of super elastic Ni-Ti alloys, and more preferably the super elastic material is nitinol. Ni-Ti alloys and in particular nitinol has excellent property compatibility with body tissue and is non-toxic.

In a preferred embodiment of the method according to the invention, the super elastic material is treated so as to have a transition temperature below 35°C. In this embodiment, the transition temperature is just below the body temperature of about 37°C, meaning that the super elastic material can be in a (more) rigid state at body temperature and in a less rigid state at a temperature below body temperature and the transition temperature as previously described.

In an alternative embodiment, the super elastic material of the first part has a transition temperature below 35°C, preferably below 28°C, and the super elastic material of the second part has a transition temperature below 50 °C. With this embodiment it is possible for the second part of the marker to be in an environment with elevated temperature but still below its transition temperature.

The treatment, to which the super elastic material is subjected to obtain its properties in respect of rigidity and optional transition temperatures, must be carefully selected, and preferably the treatment is a thermal treatment. More preferably the treatment is a thermal treatment in a furnace and/or a salt-bath, which both provide an opportunity for precise temperature control, which again gives an opportunity for optimising the thermal treatment.

In general it is preferred that the first part and the second part of the marker is subjected to the same thermal treatment. Such a procedure of treatment gives the most simple and cost-effective manufacture of the marker according to the invention. In the case of nitinol, the super elastic alloy may be subjected to a thermal treatment to gain a transition temperature in the range of 28-35°C and, consequently, the first part of the marker will be in a austenitic phase when anchored in body tissue having an temperature of approximately 37°C, while the second part extending from the body will be at room-temperature, e.g. 23°C, and in a martensitic phase, in which the alloy is softer (or in other words has a lower bending stiffness).

For some purposes it is preferred that the first part and the second part of the marker are subjected to different thermal treatments. For example, the super elastic material of the first part may be treated to have a transition temperature in the range of 28-35°C, and the superelastic material of the second part may be treated to have a transition temperature in the range of 40-50°C. Thus, in case of nitinol, a second part that will not transform from the martensitic phase to the austenitic phase at temperatures up to 50°C, but remain in the softer martensitic phase, can be obtained. Therefore, such a marker manufactured according to the method is useable at temperatures exceeding room temperature, e.g. 25-35°C, while still maintaining the second part in a softer phase.

However, it is also possible to control the transition temperature by other means than thermal treatment. Consequently, in an embodiment of the method according to the invention the first part and/or the second part is subjected to a treatment comprising treatment by cold working.

Moreover, the super elastic material may be subjected to a combination of both cold working treatment and heat treatment in order to obtain the desired properties. However, suitable treatment for a specific super elastic material can be determined by a skilled person from the specific data of the material.

In a preferred embodiment of the method at least a part of the first part is further treated to have barbs by cutting, e.g. by laser-cutting or by winding wires around the first part of the rod. The wires may be super elastic material or stainless steel or other similar materials. The barbs serve to secure a good anchoring of the marker in body tissue.

### Brief Description of the Drawings

Fig. 1 shows an embodiment of the invention seen from the side.
Fig. 2 shows the embodiment of fig. 1 seen from the end.
Fig. 3 shows the embodiment of fig. 1 seen from above.
Fig. 4 shows the embodiment of fig. 1-3 in perspective.
Fig. 5 schematically shows an embodiment for providing different properties to tubular members of the super elastic material.
-- Fig. 6 schematically shows a marker according to the invention marking a lesion in a breast.

### Detailed Description

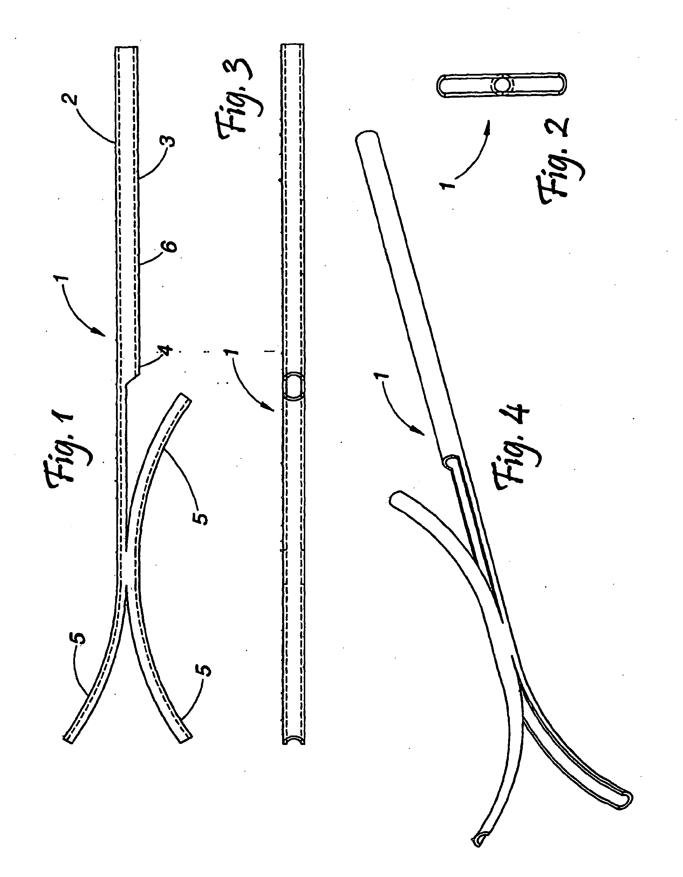

In figure 1, a marker 1 according to the invention is seen. The marker 1 is constituted by a hollow rod or tube 2 having a first part 3 and a second part 4. The first part 3 is equipped with barbs 5, which are provided by cuts in the tube 2. The border between the first part and the second part is indicated by line 6. Although the line is normally invisible, it may be indicated by a print, colour or groove on the tube 2 for use by e.g. the radiologist. When using a marker having the same transition temperature, e.g. in the range 28-35°C, throughout the material such a distinction is not necessary, while in use the part fixed in body tissue will act as the first part and the part extending from the body will act as the second part, i.e. having a lower bending stiffness.

In figure 2 the same marker is seen from the distal end. In figure 3 the marker is seen from above, and in figure 4 a depiction is shown in perspective.

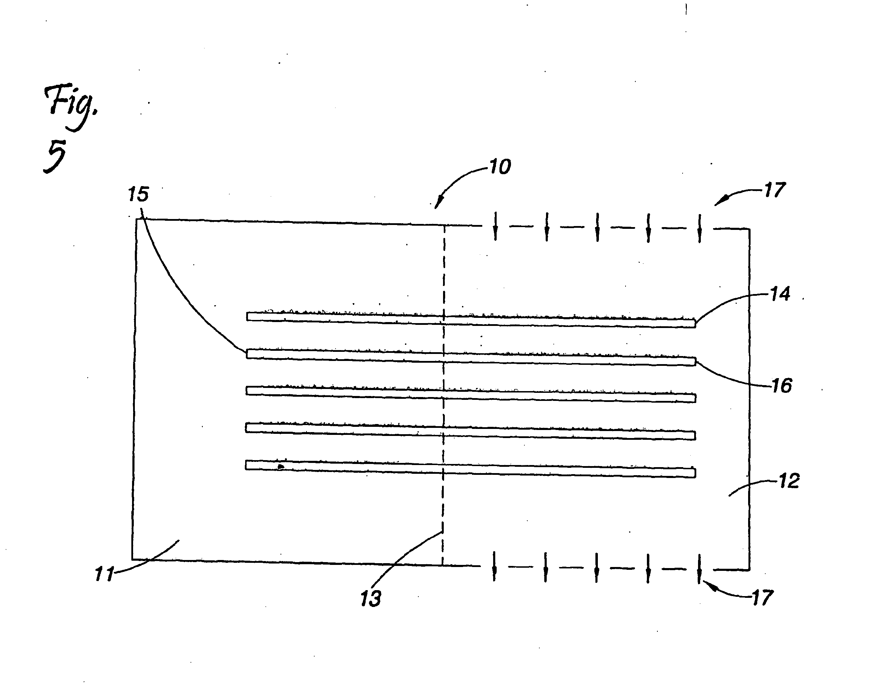

Figure 5 schematically depicts a furnace 10, which is useable for thermal treatment of the markers according to the invention. The furnace 10 comprises a first chamber 11 and a second chamber 12. The first chamber 11 is separated from the second chamber 12 by means of a wall 13. The wall 13 comprises means to hold the markers 14 to be treated in such a way that a part 15 of the marker is in the first chamber 11 and an other part 16 is in the second chamber 12. The part 15 of the markers present in the first chamber 11 can be subjected to heating while the part 16 of the markers in the second chamber 12 can be subjected to cooling (or heating at lower temperature) by a stream of fluid 17.

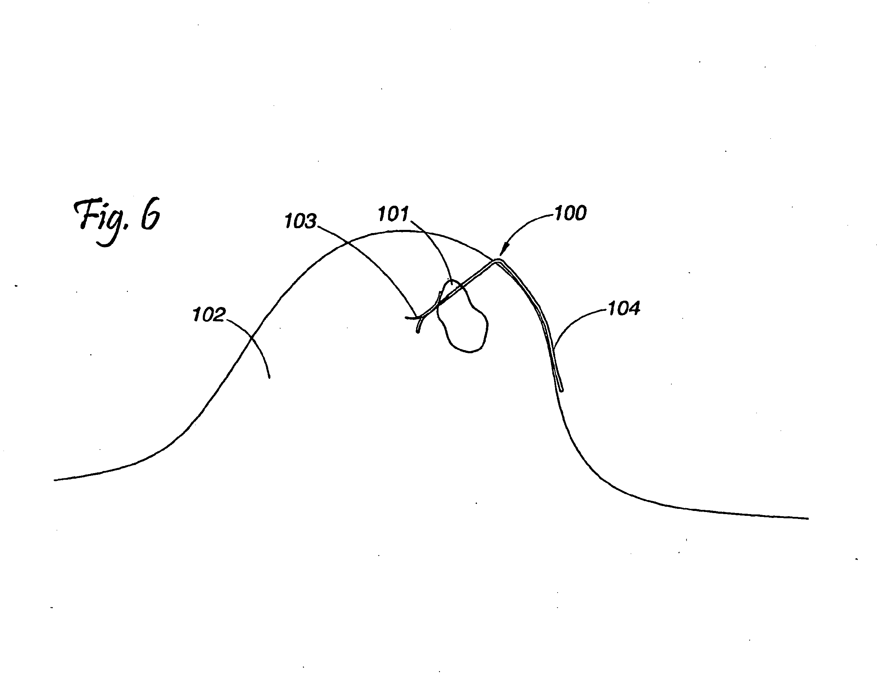

Figure 6 schematically depicts the marker 100 marking a lesion 101 in a breast 102. The rigid first part 103 of the marker 100 penetrates the lesion 101 in the breast 102 thereby marking the lesion for surgical treatment. The part of the marker 104 extending from the breast 102 is in a softer state, i.e. has lower bending stiffness, and substantially follows the curves of the breast 102. Consequently, the possibility of any blow or push on the part of the marker 104 extending from the breast is substantially eliminated.

The different values of bending stiffness of the markers according to the invention may be determined by using a Tinius Olsen stiffness tester in accordance with e.g. ASTM E855 method A. However for the purpose of having a quick and an uncomplicated method for estimating the bending stiffness of the first part of the marker in relation to the second part of the marker, the following test method was established.

### Example

A number of markers corresponding to the marker depicted in figure 1 to 4 were manufactured for test purposes.

The markers were manufactured from nitinol tubes (nitinol alloy SE508 Tubing, obtained from NDC, Germany) with an outer diameter of 0.25 mm and an inner diameter of approx. 0.20 mm. The tubes delivered from NDC had an overall transition temperature below 18°C. The tubes were cut to have a length of 10.00 cm and divided in a first part (5.0 cm) and a second part (5.0 cm). The second part was subjected to the following thermal treatment (performed by ADMEDES Schuessler, Germany):
the second part was placed in a salt bath
and heated to 540-550°C for approx. 20 hours, while the first part was kept as close to room temperature as possible

After the thermal treatment, the second part of the nitinol tubes had a transition temperature in the range of about 28-32°C. Thus, above 32°C the second part of the tubes was in an austenitic phase with a relatively high bending stiffness. Below 28°C the second part of the tubes was in a martensitic phase with a significantly lower bending stiffness. The first parts of the tubes maintained a transition temperature below 18°C and appeared rather stiff.

To improve the fastening properties in body tissue, barbs were provided in the first part of the tube intended to constitute the first part of the marker. The barbs were made by laser-cutting pieces in the tube to form "tongue-like" members (as seen in fig. 1-4).

The bending stiffness of the markers (first part in relation to the second part) was examined. Measurements of bending stiffness on the manufactured markers were carried out as follows:

The markers to be examined for bending stiffness were placed on a horizontal table with a substantially friction-free surface.

Initially, each marker had the first part fixed in a tubular holder attached to the table in such a manner that the second part could move freely on the table in horizontal directions.

A probe (stainless steel rod, diameter 2 mm) connected to a dynamometer (MARK 10) was placed horizontal on the table and perpendicular to the second part in such a way that the end of the probe contacted the second part of the marker 2.5 cm from the fixing point of the marker. The probe was then moved 0,5 cm in the direction perpendicular to the extension of the marker while forcing the second part of the marker to bend 0.5 cm at the contact point. The forced applied on the second part was then measured with the dynamometer.

The procedure was then repeated on the first part of the marker while the second part was fixed to the table. The temperature during the measurements was 20-23°C.

The results of the measurement are given in table 1.

**Table 1. Results of measurements of bending stiffness of markers according to the invention**

| | | |
|---|---|---|
| | First part | Second part |
| | (Average of measurements on 5 markers) | (Average of measurements on 5 markers) |
| Force applied N | 0.528 | 0.027 |

The results of the measurements clearly demonstrate that the bending stiffness of the markers in the first part was significantly higher than for the second part.

## Claims

1. A marker (1) for marking an area in body tissue, said marker (1) being substantially rod-shaped and being formed of a super-elastic alloy, preferably a NiTi-alloy, having a more rigid phase above a transition temperature and a less rigid phase below the transition temperature, the marker (1) comprising:
a first part (3) having anchoring means (5) intended to anchor the first part in body tissue in a body with the super-elastic alloy in said first part having a transition temperature below 35°C such that the first part (3) is in said more rigid phase at body temperature, and
a second part (4) intended to substantially extend from the body while said first part (3) is anchored in body tissue with the super-elastic alloy in said second part (4) having a transition temperature which is the same as or greater than the transition temperature of the super-elastic alloy in said first part (3),
wherein the bending stiffness of said first part (3) is at least twice as high as the bending stiffness of said second part (4) when said first part is anchored in body tissue.

2. The marker of claim 1, wherein the bending stiffness of said first part (3) is at least four, preferably eight and still more preferably twenty times as high as the bending stiffness of said second part (4) when said first part is anchored in body tissue.

3. The marker of claim 1or claim 2, wherein the super-elastic alloy is selected from the group consisting of NiTi alloys comprising 47-58% Ti.

4. The marker of any one of the preceding claims, wherein the super-elastic alloy of the first part (3) has a transition temperature in the range of 28-35°C, and the super-elastic alloy of the second part (4) has a transition temperature in the range of 50-60°C.

5. The marker of any one of the preceding claims, wherein at least a part of the first part (3) of the marker (1) is barbed.

6. The marker of any one of the preceding claims, wherein the marker (1) has a length in the range of 2 to 30 cm.

7. The marker of any one of the preceding claims, wherein the first part (3) of the marker (1) has a length in the range of 1 to 14 cm.

8. The marker of any one of the preceding claims, wherein the marker (1) has a circular cross-section with a diameter in the range of 0.25 to 1.5 mm.

9. The marker of any one of the preceding claims, wherein said marker (1) comprises a hollow rod (2).

10. A method for producing a marker for marking (1) an area in body tissue, said method comprising the steps of:
providing a rod-shaped member (2) of a super-elastic alloy and preferably a NiTi alloy, and
subjecting the marker (1) to a treatment to provide a first part (3) with a transition temperature below 35°C and a second part (4) with a transition temperature which is the same as or greater than the transition temperature of the first part (3),
such that the bending stiffness of said first part (3) is at least twice, preferably ten times and still more preferably twenty times as high as the bending stiffness of said second part (4) when said first part (3) is held at a temperature in the range of 36-39°C.

11. The method of claim 10, wherein the first and second parts (3, 4) are subjected to differential treatment.

12. The method of claim 10 or claim 11, comprising the further step of providing said first part (3) with anchoring means (5).

13. The method of any one of claims 10 to 12, wherein the super-elastic alloy is selected from the group consisting of NiTi alloys comprising 47-58% Ti.

14. The method of any one of claims 10 to 13, wherein the super-elastic alloy of the first part (3) is subjected to a treatment to provide a transition temperature below 35°C, and the material of the second part (4) is subjected to a treatment to provide a transition temperature in the range of 50-60°C.

15. The method of any one of claims 10 to 14, wherein at least a part of the first part (3) is barbed by cutting, e.g. by laser-cutting or by winded wires.

## Patentansprüche

1. Marker (1) zur Markierung eines Bereichs in einem Körpergewebe, wobei der Marker (1) im Wesentlichen stangenförmig ist und aus einer superelastischen Legierung, vorzugsweise einer NiTi-Legierung, besteht, die oberhalb einer Übergangstemperatur eine starrere Phase und unterhalb der Übergangstemperatur eine weniger starre Phase aufweist, wobei der Marker (1) Folgendes umfasst: einen ersten Teil (3) mit einem Ankermittel (5) zur Verankerung des ersten Teils in Körpergewebe in einem Körper, wobei die superelastische Legierung im ersten Teil eine Übergangstemperatur unter 35°C aufweist, so dass sich der erste Teil (3) bei Körpertemperatur in der starreren Phase befindet, und einen zweiten Teil (4), der sich im Wesentlichen vom Körper aus erstrecken soll, während der erste Teil (3) im Körpergewebe verankert ist, wobei die superelastische Legierung im zweiten Teil (4) eine Übergangstemperatur aufweist, die gleich oder größer ist als die Übergangstemperatur der superelastischen Legierung im ersten Teil (3), wobei die Biegesteifheit des ersten Teils (3) zumindest doppelt so hoch ist wie die Biegesteifheit des zweiten Teils (4), wenn der erste Teil in Körpergewebe verankert ist.

2. Marker nach Anspruch 1, wobei die Biegesteifheit des ersten Teils (3) zumindest vier Mal, vorzugsweise acht Mal und insbesondere zwanzig Mal so hoch ist wie die Biegesteifheit des zweiten Teils (4), wenn der erste Teil in Körpergewebe verankert ist.

3. Marker nach Anspruch 1 oder 2, wobei die superelastische Legierung aus der aus NiTi-Legierungen mit 47-58% Ti bestehenden Gruppe ausgewählt ist.

4. Marker nach einem der vorhergehenden Ansprüche, wobei die superelastische Legierung des ersten Teils (3) eine Übergangstemperatur im Bereich von 28-35°C und die superelastische Legierung des zweiten Teils (4) eine Übergangstemperatur im Bereich von 50-60°C aufweisen.

5. Marker nach einem der vorhergehenden Ansprüche, wobei zumindest ein Teil des ersten Teils (3) des Markers (1) mit Widerhaken versehen ist.

6. Marker nach einem der vorhergehenden Ansprüche, wobei der Marker (1) eine Länge im Bereich von 2 bis 30 cm aufweist.

7. Marker nach einem der vorhergehenden Ansprüche, wobei der erste Teil (3) des Markers (1) eine Länge im Bereich von 1 bis 14 cm aufweist.

8. Marker nach einem der vorhergehenden Ansprüche, wobei der Marker (1) einen kreisförmigen Querschnitt mit einem Durchmesser im Bereich von 0,25 bis 1,5 mm aufweist.

9. Marker nach einem der vorhergehenden Ansprüche, wobei der Marker (1) eine hohle Stange (2) umfasst.

10. Verfahren zur Herstellung eines Markers (1) zur Markierung eines Bereichs in Körpergewebe, wobei das Verfahren die folgenden Schritte umfasst: Bereitstellung eines stangenförmigen Elements (2) aus einer superelastischen Legierung und vorzugsweise aus einer NiTi-Legierung und Behandlung des Markers (1), um einen ersten Teil (3) mit einer Übergangstemperatur unter 35°C und einen zweiten Teil (4) mit einer Übergangstemperatur, die gleich oder größer ist als die Übergangstemperatur des ersten Teils (3) zu erhalten, so dass die Biegesteifheit des ersten Teils (3) zumindest zwei Mal, vorzugsweise zehn Mal und insbesondere zwanzig Mal so hoch ist wie die Biegesteifheit des zweiten Teils (4), wenn der erste Teil bei einer Temperatur im Bereich von 36-39°C gehalten wird.

11. Verfahren nach Anspruch 10, wobei die ersten und zweiten Teile (3, 4) einer unterschiedlichen Behandlung unterzogen werden.

12. Verfahren nach Anspruch 10 oder 11, das ferner den Schritt umfasst, dass der erste Teil (3) mit einem Verankerungsmittel (5) ausgestattet wird.

13. Verfahren nach einem der Ansprüche 10 bis 12, wobei die superelastische Legierung aus der aus NiTi-Legierungen mit 47-58% Ti bestehenden Gruppe ausgewählt ist.

14. Verfahren nach einem der Ansprüche 10 bis 13, wobei die superelastische Legierung des ersten Teils (3) einer Behandlung unterzogen wird, um eine Übergangstemperatur unter 35°C zu erhalten, und wobei das Material des zweiten Teils (4) einer Behandlung unterzogen wird, um eine Übergangstemperatur im Bereich von 50-60°C zu erhalten.

15. Verfahren nach einem der Ansprüche 10 bis 14, wobei zumindest ein Teil des ersten Teils (3) durch Schneiden, z.B. Laserschneiden oder durch gewundene Drähte, mit Widerhaken versehen wird.

## Revendications

1. Marqueur (1) permettant de marquer une zone sur un tissu corporel, ledit marqueur (1) ayant sensiblement la forme d'une tige et étant réalisé dans un alliage super-élastique, de préférence un alliage en NiTi qui a une phase plus rigide au-dessus d'une température de transition et une phase moins rigide en dessous de la température de transition, le marqueur (1) comportant :
une première partie (3) munie d'un moyen (5) d'ancrage destiné à ancrer la première partie dans un tissu corporel sur un corps avec l'alliage super-élastique dans ladite première partie dont la température de transition est en dessous de 35 °C de telle sorte que la première partie (3) est dans ladite phase plus rigide à la température corporelle, et
une deuxième partie (4) destinée à s'étendre sensiblement depuis le corps tandis que ladite première partie (3) est ancrée dans le tissu corporel avec l'alliage super-élastique dans ladite deuxième partie (4) dont la température de transition est égale ou supérieure à la température de transition de l'alliage super-élastique dans ladite première partie (3)
dans lequel la rigidité à la flexion de ladite première partie (3) est au moins deux fois plus élevée que la rigidité à la flexion de ladite deuxième partie (4) lorsque ladite première partie est ancrée dans le tissu corporel.

2. Marqueur selon la revendication 1, dans lequel la rigidité à la flexion de ladite première partie (3) est au moins quatre fois, de préférence huit fois et de manière encore plus souhaitable vingt fois plus élevée que la rigidité à la flexion de ladite deuxième partie (4) lorsque ladite première partie est ancrée dans le tissu corporel.

3. Marqueur selon la revendication 1 ou la revendication 2, dans lequel l'alliage super-élastique est choisi dans le groupe constitué d'alliages en NiTi comportant 47 à 58 % de titane.

4. Marqueur seul selon l'une quelconque des revendications précédentes, dans lequel l'alliage super-élastique de la première partie (3) a une température de transition de l'ordre de 28 à 35 °C, et l'alliage super-élastique de la deuxième partie (4) a une température de transition de l'ordre de 50 à 60 °C.

5. Marqueur selon l'une quelconque des revendications précédentes, dans lequel au moins une partie de la première partie (3) du marqueur (1) a des barbillons.

6. Marqueur selon l'une quelconque des revendications précédentes, dans lequel le marqueur (1) a une longueur de l'ordre de 2 à 30 cm.

7. Marqueur selon l'une quelconque des revendications précédentes, dans lequel la première partie (3) du marqueur (1) a une longueur de l'ordre de 1 à 14 cm.

8. Marqueur selon l'une quelconque des revendications précédentes, dans lequel le marqueur (1) a une section transversale circulaire d'un diamètre de l'ordre de 0,25 à 1,5 mm.

9. Marqueur selon l'une quelconque des revendications précédentes, dans lequel ledit marqueur (1) comporte une tige creuse (2).

10. Procédé de production d'un marqueur permettant de marquer (1) une zone sur un tissu corporel, ledit procédé comportant les étapes consistant à :
fournir un élément en forme de tige (2) en un alliage super-élastique et de préférence un alliage en NiTi, et
soumettre le marqueur (1) à un traitement pour fournir une première partie (3) avec une température de transition en dessous de 35 °C et une deuxième partie (4) avec une température de transition qui est égale ou supérieure à la température de transition de la première partie (3).
de sorte que la rigidité à la flexion de ladite première partie (3) est au moins deux fois, de préférence dix fois et de manière encore plus souhaitable vingt fois plus élevée que la rigidité à la flexion de ladite deuxième partie (4) lorsque ladite première partie (3) est maintenue à une température de l'ordre de 36 à 39 °C.

11. Procédé selon la revendication 10, dans lequel les première et deuxième parties (3, 4) sont soumises à un traitement différentiel.

12. Procédé selon la revendication 10 ou la revendication 11, comportant l'étape supplémentaire consistant à fournir un moyen (5) d'ancrage à la première partie (3).

13. Procédé selon l'une quelconque des revendications 10 à 12, dans lequel l'alliage super-élastique est choisi dans le groupe constitué d'alliages en NiTi comportant 47 à 58 % de titane.

14. Procédé selon l'une quelconque des revendications 10 à 13, dans lequel l'alliage super-élastique de la première partie (3) est soumis à un traitement pour fournir une température de transition en dessous de 35 °C, et le matériau de la deuxième partie (4) est soumis à un traitement pour fournir une température de transition de l'ordre de 50 à 60 °C.

15. Procédé selon l'une quelconque des revendications 10 à 14, dans lequel au moins une partie de la première partie (3) a des barbillons obtenus par découpe, par ex. par découpe au laser ou par fils bobinés.
